(19) Europäisches Patentamt
European Patent Office
Office européen des brevets

(11) Veröffentlichungsnummer : **0 060 425**
**B1**

# (12) EUROPÄISCHE PATENTSCHRIFT

(45) Veröffentlichungstag der Patentschrift : **16.06.87**

(51) Int. Cl.⁴ : **C 07 D487/04**

(21) Anmeldenummer : **82101512.0**

(22) Anmeldetag : **27.02.82**

(54) **Glykolurilsalze und Verfahren zu deren Herstellung.**

(30) Priorität : **12.03.81 DE 3109478**

(43) Veröffentlichungstag der Anmeldung : **22.09.82 Patentblatt 82/38**

(45) Bekanntmachung des Hinweises auf die Patenterteilung : **16.06.87 Patentblatt 87/25**

(84) Benannte Vertragsstaaten : **DE FR GB IT**

(56) Entgegenhaltungen :
**DE-A- 2 229 439**
**CHEMICAL ABSTRACTS, Vol. 92, No. 11, 17. März 1980, Columbus, Ohio, USA, L.I. SUVOROVA, L.V. EPISHINA, O.V. LEBEDEV, L.I. KHMEL'NITSKI, S.S. NOVIKOV "Study of the chemistry of bicyclic bisureas. Communication 3. Synthesis and hydrolytic stability of diborofluoride salts of 3,7-diethoxy-2,6-diaza-4,8-diazonia bicyclo (3.3.o)octane-3,7-diethoxy-2,6-diaza-4,8-diazoniabicyclo(3.3.1)nonane-3,7-dienes." Seite 590, Spalte 2, Zusammenfassung No. 94365x**
**BEILSTEINS HANDBUCH DER ORGANISCHEN CHEMIE, Band 26, 1937 VERLAG JULIUS SPRINGER "Dixo-Verbindungen", Berlin Seiten 441-443**
**Römpps Chemielexikon (1981),Stichwort "Flammschutzmittel" Ullmanns Encyklopädie der techn. Chemie (4) 503 und 510-512.**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Rottmaier, Ludwig**
**Bergstrasse 85**
**D-5068 Odenthal (DE)**
Erfinder : **Merten, Rudolf, Dr.**
**Berta-von-Suttner-Strasse 55**
**D-5090 Leverkusen 1 (DE)**

**EP 0 060 425 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aus Ullmanns Encyklopädie der technischen Chemie, 4. Auflage, Bd. 16, 511 (1978) war bekannt, daß durch Umsetzung von Melamin mit anorganischen oder organischen Säuren Melaminsalze, beispielsweise Melamin-Cyanurat, erhalten werden, die besonders als Flammschutzmittel Bedeutung erlangt haben. Gegenüber Melamin-Cyanurat verleihen die erfindungsgemäßen Salze einem Thermoplasten wie beispielsweise Polyamid verbesserte mechanische Eigenschaften, wie z. B. eine höhere Kerbschlagzähigkeit.

Gegenstand der vorliegenden Erfindung sind Glykoluril-Triazin-Salze, erhältlich durch Umsetzung von Glykolurilen der Formel

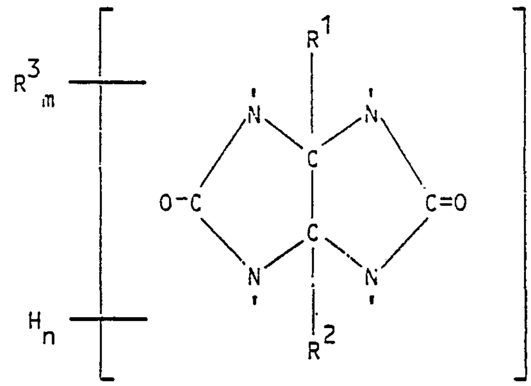

(I)

worin

$R^1$ und $R^2$ für Wasserstoff, einen aliphatischen $C_1$-$C_{20}$- oder einen aromatischen $C_6$-$C_{10}$-Kohlenwasserstoffrest,

$R^3$ für einen aliphatischen $C_1$-$C_{20}$-, einen cycloaliphatischen $C_4$-$C_{15}$-, einen araliphatischen $C_7$-$C_{15}$- oder einen aromatischen $C_6$-$C_{15}$-Kohlenwasserstoffrest,

n für eine ganze Zahl von 1 bis 4 und

m für 4 — n

stehen, mit — pro Mol Glykoluril I — mindestens 1 und höchstens n Mol Triazin der Formel

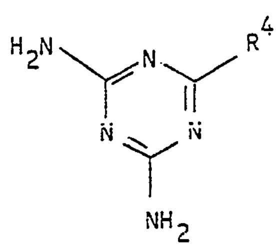

(II)

worin $R^4$ für Wasserstoff, eine Aminogruppe, einen gegebenenfalls mit Halogen (wie z. B. Fluor, Chlor oder Brom) substituierten Rest aus der Reihe aliphatischer $C_1$-$C_{20}$-, cycloaliphatischer $C_4$-$C_{17}$-, araliphatischer $C_7$-$C_{17}$- und aromatischer $C_6$-$C_{15}$-Kohlenwasserstoffrest steht.

Vorzugsweise stehen $R^1$ und $R^2$ für Wasserstoff, einen $C_1$-$C_{10}$-Alkylrest oder einen Phenylrest, insbesondere für Wasserstoff, Methyl, Ethyl, Propyl oder Butyl. Ganz besonders bevorzugt bedeuten $R^1$ und $R^2$ Wasserstoff oder eine Methylgruppe.

Vorzugsweise steht $R^3$ für einen aliphatischen $C_1$-$C_{10}$-, einen cycloaliphatischen $C_4$-$C_{10}$-, einen araliphatischen $C_7$-$C_{10}$- oder einen aromatischen $C_6$-$C_{10}$-Kohlenwasserstoffrest, insbesondere für einen Methyl-, Ethyl-, Propyl-, Butyl-, Cyclohexyl-, Benzyl- oder Phenylrest. Ganz besonders bevorzugt bedeutet $R^3$ eine Methylgruppe.

Der Rest $R^4$ ist vorzugsweise unsubstituiert und steht vorzugsweise für Wasserstoff, eine Aminogruppe, eine aliphatischen $C_1$-$C_{10}$-, einen cycloaliphatischen $C_4$-$C_{10}$-, einen araliphatischen $C_7$-$C_{10}$- oder einen aromatischen $C_6$-$C_{10}$-Kohlenwasserstoffrest, besonders bevorzugt für Wasserstoff, eine Aminogruppe, einen aliphatischen $C_1$-$C_6$-, einen cycloaliphatischen $C_4$-$C_6$- einen Benzyl- oder Phenylrest. Ganz besonders bevorzugt steht $R^4$ für die Aminogruppe.

Die für die Herstellung der erfindungsgemäßen Salze notwendigen Ausgangsglykolurile können nach bekannten Verfahren aus α,β-Diketoverbindungen und Harnstoffen, gegebenenfalls in Gegenwart von sauren Katalysatoren, hergestellt werden. Die Herstellung der Glykolurile kann z. B. analog des Vorschrift, wie in Liebigs Annalen 189, (1877) S. 157 beschrieben, erfolgen.

Die für die Herstellung der erfindungsgemäßen Salze notwendigen Ausgangstriazine können nach bekannten Verfahren, z. B. aus Nitrilen und Dicyandiamin in polaren organischen Lösungsmitteln, wie z.

B. Dimethylsulfoxid, analog Bulletin of the Chemical Society, Japan 38, Nr. 11 (1965), 1820 hergestellt werden.

Die Reaktion der Glykolurile mit den Triazinen wird normalerweise mit äquivalenten Mengen durchgeführt, unter Berücksichtigung des Umstandes, dass eine NH-Gruppe des Glykolurils mit einem Triazin zur Reaktion gebracht werden kann so daß also Glykolurile der allgemeinen Formel (I), in denen m = 0 und n = 4 bedeutet, mit 4 Mol Triazin reagieren können.

Es ist aber durchaus möglich, die soeben genannten Glykolurile mit z. B. 2 Mol Triazin umzusetzen, so daß noch freie NH-Gruppen in den erfindungsgemäßen Salzen enthalten sind.

Die Reaktion zwischen den Glykolurilen und den Triazinen kann in der Schmelze oder — vorzugsweise — in Lösungsmitteln durchgeführt werden.

Als Lösungsmittel können mit den Reaktionskomponenten nicht reagierende, insbesondere polare, Lösungsmittel verwendet werden.

Vorteilhafterweise wird die Reaktion zwischen den Glykolurilen und den Triazinen jedoch in wäßrigem Medium und/oder in mit Wasser mischbaren Lösungsmittel durchgeführt. Als Beispiele für diese mit Wasser mischbaren Lösungsmittel seien genannt : Alkohole wie Methanol, Ethanol, Isopropanol, Glykolmonomethylether und Glykol, cyclische Ether wie Tetrahydrofuran und Dioxan, Alkyl- und Dialkylamide wie Dimethylformamid, Dimethylacetamid und N-Methylpyrrolidon. Selbstverständlich können auch Mischungen mit Wasser verwandt werden. Auch können nach beendeter Reaktion, evtl. zur besseren Aufarbeitung der erhaltenen Glykoluril-Salze, weitere Lösungsmittel, wie z. B. aliphatische und aromatische Kohlenwasserstoffe (Ligroin, Cyclohexan, Toluol, Xylol), chlorierte Kohlenwasserstoffe (Methylenchlorid, Chloroform, Chlorbenzol) und Ester (Essigsäureethyl-, -butylester) zugesetzt werden.

Insbesondere findet bei der Reaktion zwischen den Glykolurilen und den Triazinen Wasser als Lösungsmittel Verwendung, da häufig die gewünschte Verbindung in praktisch reiner Form aus der wäßrigen Lösung auskristallisiert.

Die Salzbildung zwischen den Glykolurilen und den Triazinen wird bei 0 bis 180 °C, vorzugsweise bei 30 bis 130 °C, gegebenenfalls unter erhöhtem Druck, durchgeführt.

Die Reaktionszeiten liegen im allgemeinen zwischen einigen Minuten und mehreren Stunden, können in besonderen Fällen jedoch auch darüber liegen. Durch entsprechende Wahl der Reaktionsbedingungen, z. B. Druck, werden kürzere Reaktionszeiten erreicht.

Die erfindungsgemäßen Glykoluril-Triazin-Salze sind wertvolle Hilfsstoffe zur Modifizierung von polymeren Substanzen. Sie können zur Herstellung und Modifizierung von Polyurethanen oder zur Flammschutzausrüstung von thermoplastischen Polyamiden eingesetzt werden.

In den nachfolgenden Beispielen bedeuten Prozente Gewichtsprozente und Teile Gewichtsteile.

## Beispiel 1

3,55 kg Glykoluril werden bei 95 °C unter Rühren zu einer Mischung aus 12,6 kg Melamin und 75 kg Wasser gegeben. Zur Vervollständigung der Reaktion wird 3 Stunden bei leichtem Rückfluß gerührt. Bei 80 °C wird der erhaltene Niederschlag abgesaugt, mit heißem Wasser gewaschen und bei 100 °C in einem Umlufttrockenschrank bis zur Gewichstskonstanz getrocknet. Es werden 13,1 kg eines Salzes aus Glykoluril und im Molverhältnis 1 : 4 erhalten, dessen Struktur mittels IR-Spektrum und der Elementaranalyse bewiesen wird.

$C_{16}H_{30}N_{28}O_2$ Ber. : C = 29,72 % H = 4,68 % N = 60,66 %
(646,6) Gef. : C = 29,5 % H = 4,7 % N = 60,4 %

## Beispiel 2

a) Einer Mischung aus 12,6 kg Melamin und 75 kg Wasser werden bei 95 °C 7,1 kg Glykoluril zugesetzt. Zur Vervollständigung der Reaktion wird 3 Stunden bei 95 °C gerührt und heiß abgesaugt, mit Wasser gewaschen und bei 100 °C in einem Umlufttrockenschrank getrocknet. Es werden 15,75 kg eines Salzes aus Glykoluril und Melamin im Molverhältnis 1 : 2 erhalten, dessen Struktur durch das IR-Spektrum und der Elementaranalyse bestätigt wird.

$C_{10}H_{18}N_{16}O_2$ Ber. : C = 30,45 % H = 4,60 % N = 56,83 %
(394,4) Gef. : C = 30,4 % H = 4,7 % N = 56,9 %

b) 92,5 Gew.-% Polyamid-6 mit einer relativen Viskosität (gemessen an einer Lösung aus 1 g Polyamid in 100 ml m-Kresol bei 25 °C) von 2,9 werden in einem Doppelwellenextruder bei den für Polyamid-6 üblichen Bedingungen mit 7,5 Gew.-% des nach a) hergestellten Melamin-Glykoluril-Salzes in der Schmelze vermischt. Der abgezogene Strang wird gekühlt, granuliert und getrocknet. Anschließend wird das Granulat auf einer Spritzgießmaschine A 270 der Firma Arburg zu Prüfkörpern nach ASTM von 1/4, 1/8 und 1/16 Zoll gespritzt.

Diese Probekörper werden 7 Tage im Trockenschrank bei 70 °C gelagert und anschließend der Prüfung gemäß Underwriter's Laboratories (UL) Subject 94 « Vertical Burning Test für Classifying Materials » unterzogen. Bei allen Prüfkörpern wurde eine Klassifizierung von VO erreicht, während das Polyamid-6 ohne Melamin-Glykoluril-Salz mit V2 getestet wurde.

Beispiel 3

Zu einer Mischung von 50,4 g Melamin und 300 g Wasser werden unter Rühren bei 95 °C 17 g 3a,6a-Dimethyl-glykoluril gegeben und 3 Stunden bei 95 °C gerührt. Der kristalline Niederschlag wird heiß abgesaugt, mit Wasser gewaschen und bei 80 °C im Vakuum bei 30 mbar getrocknet. Es werden 58,2 g eines Salzes aus Glykolurilderivat und Melamin im Molverhältnis 1 : 4 erhalten, dessen Struktur mittels IR-Spektrum und der Elementaranalyse bestätigt wird.

$C_{18}H_{34}N_{28}O_2$ Ber.: C = 32,04 % H = 5,08 % N = 58,13 %
(674,7) Gef.: C = 32,2 % H = 5,2 % N = 58,0 %

Beispiel 4

Analog Beispiel 3 werden 50,4 g Melamin und 34 g 3a,6a-Dimethyl-glykoluril in 300 g Wasser umgesetzt. Nach dem Trocknen werden 73,2 g eines Salzes aus Glykolurilderivat und Melamin im Molverhältnis 1 : 2 erhalten, dessen Struktur mittels IR-Spektrum und der Elementaranalyse bestätigt wird.

$C_{12}H_{22}N_{16}O_2$ Ber.: C = 34,12 % H = 5,25 % N = 53,06 %
(422,4) Gef.: C = 34,0 % H = 5,3 % N = 53,2 %

Beispiel 5

Analog Beispiel 3 werden 50,4 g Melamin und 34 g 1,4-Dimethyl-glykoluril in 300 g Wasser umgesetzt. Nach dem Trocknen werden 72,4 g eines Salzes aus Glykolurilderivat und Melamin im Molverhältnis 1 : 2 erhalten, dessen Struktur mittel IR-Spektrum und der Elementaranalyse bestätigt wird.

$C_{12}H_{22}N_{16}O_2$ Ber.: C = 34,12 % H = 5,25 % N = 53,06 %
(422,4) Gef.: C = 33,9 % H = 5,1 % N = 53,3 %

**Patentansprüche**

1. Glykoluril-Triazin-Salze, erhältlich durch Umsetzung von Glykolurilen der Formel

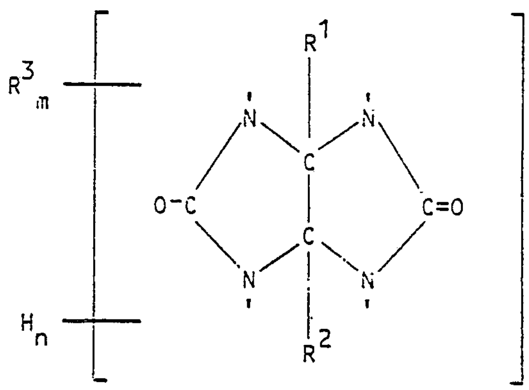

(I)

worin

$R^1$ und $R^2$ für Wasserstoff, einen aliphatischen $C_1$-$C_{20}$- oder einen aromatischen $C_6$-$C_{10}$-Kohlenwasserstoffrest,

$R^3$ für einen aliphatischen $C_1$-$C_{20}$-, einen cycloaliphatischen $C_4$-$C_{15}$-, einen araliphatischen $C_7$-$C_{15}$- oder einen aromatischen $C_6$-$C_{15}$-Kohlenwasserstoffrest,

n für eine ganze Zahl von 1 bis 4 und

m für 4 — n

stehen, mit — pro Mol Glykoluril I — mindestens und höchstens n Mol Triazin der Formel

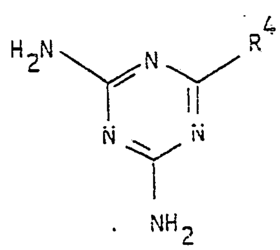

(II)

worin $R^4$ für Wasserstoff, eine Aminogruppe, einen gegebenenfalls mit Halogen substituierten Rest aus

der Reihe aliphatischer $C_1$-$C_{20}$-, cycloaliphatischer $C_4$-$C_{17}$-, araliphatischer $C_7$-$C_{17}$- und aromatischer $C_6$-$C_{15}$-Kohlenwasserstoffrest steht.

2. Salze nach Anspruch 1, dadurch gekennzeichnet, daß $R^1$ und $R^2$ für Wasserstoff, einen $C_1$-$C_{10}$-Alkylrest oder einen Phenylrest stehen.

3. Salze nach Ansprüchen 1 und 2, dadurch gekennzeichnet, daß $R^3$ für einen aliphatischen $C_1$-$C_{10}$-, einen cycloaliphatischen $C_4$-$C_{10}$-, einen araliphatischen $C_7$-$C_{10}$ oder einen aromatischen $C_6$-$C_{10}$-Kohlenwasserstoffrest steht.

4. Salze nach Ansprüchen 1 bis 3, dadurch gekennzeichnet, daß $R^4$ für Wasserstoff, eine Aminogruppe, einen aliphatischen $C_1$-$C_{10}$-, einen cycloaliphatischen $C_4$-$C_{10}$-, einen araliphatischen $C_7$-$C_{10}$- oder einen aromatischen $C_6$-$C_{10}$-Kohlenwasserstoffrest steht.

5. Salze nach Ansprüchen 1 bis 4, dadurch gekennzeichnet, daß $R^1$ und $R^2$ für Wasserstoff oder einen $C_1$-$C_4$-Alkylrest stehen.

6. Salze nach Ansprüchen 1 bis 5, dadurch gekennzeichnet, daß $R^3$ für einen $C_1$-$C_4$-Alkylrest, einen Cyclohexyl-, Benzyl- oder Phenylrest steht.

7. Salze nach Ansprüchen 1 bis 6, dadurch gekennzeichnet, daß $R^4$ für Wasserstoff, eine Aminogruppe, einen aliphatischen $C_1$-$C_6$-Kohlenwasserstoffrest, einen cycloaliphatischen $C_4$-$C_6$-Kohlenwasserstoffrest einen Benzyl- oder einen Phenylrest steht.

8. Salze nach Ansprüchen 1 bis 7, dadurch gekennzeichnet, daß
$R^1$ und $R^2$ für Wasserstoff oder eine Methylgruppe,
$R^3$ für eine Methylgruppe und
$R^4$ für eine Aminogruppe stehen.

9. Verfahren zur Herstellung von Glykoluril-Triazin-Salzen nach Ansprüchen 1 bis 8, dadurch gekennzeichnet, daß man Glykoluril der Formel (I) mit — pro Mol Glykoluril — mindestens 1 und höchstens n Mol Triazin der Formel (II) in Wasser und/oder in organischen Lösungsmitteln bei Temperaturen von 0 bis 180 °C umsetzt, wobei die Formeln (I) und (II) die in Anspruch 1 angegebene Bedeutung haben.

**Claims**

1. Glycoluril-triazine salts, obtainable by reacting glycoluril- of the formula

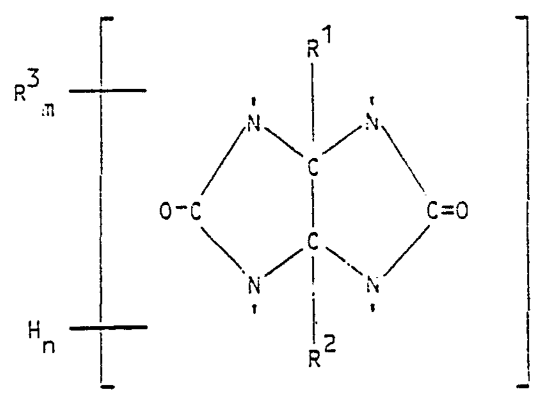

(I)

wherein
$R^1$ and $R^2$ represent hydrogen, an aliphatic $C_1$-$C_{20}$ or an aromatic $C_6$-$C_{10}$ hydrocarbon radical,
$R^3$ represents an aliphatic $C_1$-$C_{20}$, a cycloaliphatic $C_4$-$C_{15}$, an araliphatic $C_7$-$C_{15}$ or an aromatic $C_6$-$C_{15}$ hydrocarbon radical,
n represents an integer from 1 to 4 and
m represents 4 — n,
with — per mole of glycoluril I — at least 1 and at most n moles of triazine of the formula

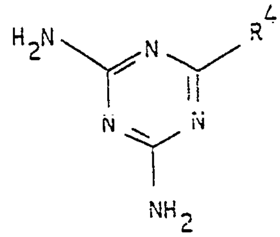

(II)

wherein $R^4$ represents hydrogen, an amino group, an optionally halogen-substituted radical from the

series comprising an aliphatic $C_1$-$C_{20}$, cycloaliphatic $C_4$-$C_{17}$, araliphatic $C_7$-$C_{17}$ and aromatic $C_6$-$C_{15}$ hydrocarbon radical.

2. Salts according to Claim 1, characterised in that $R^1$ and $R^2$ represent hydrogen, a $C_1$-$C_{10}$-alkyl radical or a phenyl radical.

3. Salts according to Claims 1 and 2, characterized in that $R^3$ represents an aliphatic $C_1$-$C_{10}$, a cycloaliphatic $C_4$-$C_{10}$, an araliphatic $C_7$-$C_{10}$ or an aromatic $C_6$-$C_{10}$ hydrocarbon radical.

4. Salts according to Claims 1 to 3, characterised in that $R^4$ represents hydrogen, an amino group, an aliphatic $C_1$-$C_{10}$, a cycloaliphatic $C_4$-$C_{10}$, an araliphatic $C_7$-$C_{10}$ or an aromatic $C_6$-$C_{10}$ hydrocarbon radical.

5. Salts according to Claims 1 to 4, characterised in that $R^1$ and $R^2$ represent hydrogen or a $C_1$-$C_4$ alkyl radical.

6. Salts according to Claims 1 to 5, characterised in that $R^3$ represents a $C_1$-$C_4$ alkyl radical, or a cyclohexyl, benzyl or phenyl radical.

7. Salts according to Claims 1 to 6, characterised in that $R^4$ represents hydrogen, an amino group, an aliphatic $C_1$-$C_6$ hydrocarbon radical, a cycloaliphatic $C_4$-$C_6$ hydrocarbon radical, or a benzyl or a phenyl radical.

8. Salts according to Claims 1 to 7, characterized in that
$R^1$ and $R^2$ represent hydrogen or a methyl group,
$R^3$ represents a methyl group and
$R^4$ represents an amino group.

9. Process for the preparation of glycoluril-triazine salts according to Claims 1 to 8, characterised in that glycoluril of the formula (I) is reacted with — per mole of glycoluril — at least 1 and at most n moles of triazine of the formula (II) in water and/or in organic solvents at temperatures of 0 to 180 °C, formulae (I) and (II) having the meaning given in Claim 1.

## Revendications

1. Sels de triazines de glycolurils, obtenus par réaction de glycolurils de formule

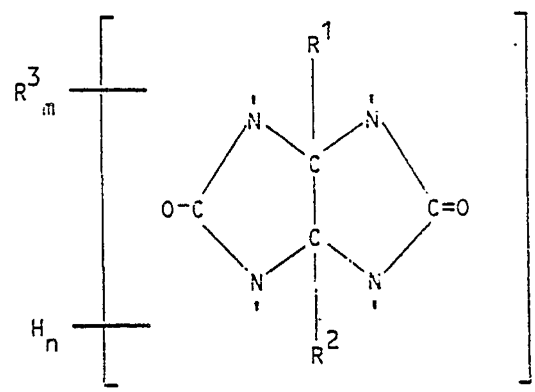

(I)

dans laquelle
$R^1$ et $R^2$ représentent l'hydrogène ou un reste d'hydrocarbure aliphatique en $C_1$-$C_{20}$ ou un reste d'hydrocarbure aromatique en $C_6$-$C_{10}$,
$R^3$ représente un reste d'hydrocarbure aliphatique en $C_1$-$C_{20}$, un reste d'hydrocarbure cycloaliphatique en $C_4$-$C_{15}$, un reste d'hydrocarbure araliphatique en $C_7$-$C_{15}$ ou un reste d'hydrocarbure aromatique en $C_6$-$C_{15}$,
n est un nombre entier de 1 à 4 et
m est égal à 4 — n,
avec au moins 1 et au plus n moles — par mole de glycolurile I — d'une triazine de formule

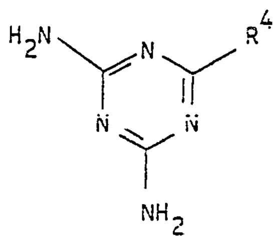

(II)

dans laquelle $R^4$ représente l'hydrogène ou un groupe amino, ou un reste éventuellement halogéno

substitué de la série des hydrocarbures aliphatiques en $C_1$-$C_{20}$, des hydrocarbures cycloaliphatiques en $C_4$-$C_{17}$, des hydrocarbures araliphatiques en $C_7$-$C_{17}$ et des hydrocarbures aromatiques en $C_6$-$C_{15}$.

2. Sels selon la revendication 1, caractérisés en ce que $R^1$ et $R^2$ représentent l'hydrogène ou un reste alkyle en $C_1$-$C_{10}$ ou un reste phényle.

3. Sels selon les revendications 1 et 2, caractérisés en ce que $R^3$ représente un reste d'hydrocarbure aliphatique en $C_1$-$C_{10}$, un reste d'hydrocarbure cycloaliphatique en $C_4$-$C_{10}$, un reste d'hydrocarbure araliphatique en $C_7$-$C_{10}$ ou un reste d'hydrocarbure aromatique en $C_6$-$C_{10}$.

4. Sels selon les revendications 1 à 3, caractérisés en ce que $R^4$ représente l'hydrogène, un groupe amino, un reste d'hydrocarbure aliphatique en $C_1$-$C_{10}$, un reste d'hydrocarbure cycloaliphatique en $C_4$-$C_{10}$, un reste d'hydrocarbure araliphatique en $C_7$-$C_{10}$ ou un reste d'hydrocarbure aromatique en $C_6$-$C_{10}$.

5. Sels selon les revendications 1 à 4, caractérisés en ce que $R^1$ et $R^2$ représentent l'hydrogène ou un reste alkyle en $C_1$-$C_4$.

6. Sels selon les revendications 1 à 5, caractérisés en ce que $R^3$ représente un reste alkyle en $C_1$-$C_4$, un reste cyclohexyle, un reste benzyle ou un reste phényle.

7. Sels selon les revendications 1 à 6, caractérisés en ce que $R^4$ représentent l'hydrogène ou un groupe amino, un reste d'hydrocarbure aliphatique en $C_1$-$C_6$, un reste d'hydrocarbure cycloaliphatique en $C_4$-$C_6$ ou un reste phényle.

8. Sels selon les revendications 1 à 7, caractérisés en ce que
$R^1$ et $R^2$ représentent l'hydrogène ou un groupe méthyle,
$R^3$ représente un groupe méthyle et
$R^4$ représente un groupe amino.

9. Procédé pour la fabrication de sels de triazines de glycolurilles selon les revendications 1 à 8, caractérisé en ce que l'on fait réagir un glycolurile de formule (I) avec au moins 1 et au plus n moles — par mole de glycolurile — d'une triazine de formule (II) à des températures de 0-80 °C dans l'eau et/ou dans des solvants organiques, les formules (I) et (II) ayant la signification indiquée dans la revendication 1.